# EUROPEAN PATENT SPECIFICATION

(11) **EP 3 537 152 B1**
(45) Date of publication and mention of the grant of the patent: **14.06.2023**
(21) Application number: 17861585.2
(22) Date of filing: 17.10.2017
(51) Int. Cl.: G01N 33/53, C07K 16/40, G01N 33/573

(54) **IMMUNOASSAY METHOD FOR DETERMINING RENIN CONCENTRATION**
IMMUNOASSAY-VERFAHREN ZUR BESTIMMUNG DER RENINKONZENTRATION
MÉTHODE D'IMMUNOESSAI PERMETTANT DE DÉTERMINER UNE CONCENTRATION DE RÉNINE

(30) Priority: 21.10.2016 JP 2016206957
(43) Date of publication of application: 11.09.2019
(73) Proprietor: Fujirebio Inc., Shinjuku-ku Tokyo 163-0410 (JP)
(72) Inventor: SAKYU Takuya, Tokyo 163-0410 (JP); HAMANO Kumiko, Tokyo 163-0410 (JP); HARA Ryujiro, Tokyo 163-0410 (JP)
(74) Representative: Müller-Boré & Partner Patentanwälte PartG mbB
(86) International application number: PCT/JP2017/037484
(87) International publication number: WO 2018/074455

(56) References cited:
- JP-A- H0 892 299
- JP-A- S6 485 070
- US-A1- 2010 196 367
- JACOB BOUHNIK ET AL: "Production and Characterization of Human Renin Antibodies with Region-oriented Synthetic Peptides*", THE JOURNAL OF BIOLOGICAL CHEMISTRY, vol. 262, no. 6, 25 February 1987 (1987-02-25), pages 2913-2918, XP055689862,
- ZUO, WM ET AL.: 'Characterization of a Monoclonal Antibody Specific for Human Active Renin' HYPERTENSION vol. 19, no. 3, March 1992, pages 249 - 254, XP005478094
- BLUNDELL, T ET AL.: 'Three-dimensional structure, specificity and catalytic mechanism of renin' NATURE vol. 304, no. 5923, 21 July 1983, pages 273 - 275, XP055478101

## Description

### [Technical Field]

The present invention relates to an antibody specific to active renin, an immunological measurement method for renin concentration using the antibody, a drug containing the antibody, and a kit containing the drug.

### [Background Art]

Renin is a protein which regulates blood pressure with the renin-angiotensin-aldosterone system, and its activity is an important index in the diagnosis of e.g. hypertension and primary aldosteronism.

In the above context, methods for measuring renin activity and renin concentration have been developed, and the current mainstream in Japan is a method for measuring renin activity which detects angiotensin-I produced when the renin in plasma degrades angiotensinogen (NPL 1). However, this method has a problem that the renin activity depends on the concentration of angiotensinogen in plasma.

On the other hand, a method for detecting active renin is also used as a method which is not affected by the concentration of angiotensinogen. Mature renin, which is active, is produced from prorenin, and the prorenin is classified into two forms of closed structure and open structure, of which the open structure is active. Therefore, in the measurement of active renin, methods capable of immunologically measuring both mature renin and open structure prorenin have also been developed (NPL 1) .

However, the antibodies used in those immunological methods recognize mature renin or prorenin inactivated by binding of a renin inhibitor (NPLs 2 and 3). This thus poses a problem that the measured renin concentration does not correlate with renin activity in the case of measuring the active renin of a patient to whom a renin inhibitor has been administered.

### [Citation List]

### [Non Patent Literature]

[NPL 1] Campbell DJ et al. Clin Chem. 2009 May; 55 (5) : 867-77
[NPL 2] Campbell DJ. Hypertension. 2008 Jan; 51 (1): 15-8
[NPL 3] Derkx FH. Clin Chem. 1996 Jul; 42 (7): 1051-63

### [Summary of Invention]

### [Technical Problem]

The present invention has been made in view of the above circumstances, and an object thereof is to provide a method for immunologically measuring a renin concentration, in which the measured renin concentration correlates with renin activity even in the presence of a renin inhibitor. Also, the present invention aims to provide an active renin-specific antibody for the measurement, a drug containing the active renin-specific antibody, and a kit containing the drug.

### [Solution to Problem]

For the purpose of achieving the above objects, the present inventors constructed a system for immunologically measuring the renin concentration in the presence of a renin inhibitor, and used the system to evaluate the relationship between the renin activity and the measured value of renin concentration for the selected antibodies in the presence of the renin inhibitor. As a result, the present inventors finally succeeded in identifying, among many of the antibodies evaluated, a plurality of antibodies for which the renin concentration measured in the presence of a renin inhibitor strongly correlated with renin activity.

For the identified antibodies, the measured renin concentration strongly correlated with renin activity not only in the case of adding a renin inhibitor aliskiren but also in the case of adding other renin inhibitors Renin Inhibitor III and VTP-27999. This fact means that the acquired antibodies show high reactivity to renin having activity without binding of a renin inhibitor, but show a greatly reduced reactivity to renin inactivated by binding of a renin inhibitor. In addition, the antibodies which show such reactivity recognized a common epitope on renin.

On the other hand, commercially available antibodies also showed high reactivity to renin inactivated by binding of a renin inhibitor as in conventional reports, and no correlation was observed between the measured renin concentration and renin activity.

As described above, the present inventors succeeded in the world's first development of antibodies which show reactivity specific to active renin regardless of the presence or absence of a renin inhibitor. Also, the present inventors used the antibodies to succeed in constructing an immunological measurement method in which the measured renin concentration strongly correlates with renin activity. In this way, the present invention has been completed.

Specifically, the present invention relates to antibodies specific to active renin, an immunological measurement method for renin concentration using the antibodies, a drug containing the antibodies, and a kit containing the drug, and more specifically provides the following.
[1] A monoclonal antibody characterized by the following (a) to (c); (a) showing higher reactivity to mature renin and open structure prorenin, both of which are not bound with a renin inhibitor, than to mature renin and open structure prorenin, both of which are inactivated by binding of a renin inhibitor, (b) binding to a peptide composed of 30th to 86th amino acids of an amino acid sequence set forth in SEQ ID NO: 1, and (c) not binding to a peptide composed of 58th to 170th amino acids of an amino acid sequence set forth in SEQ ID NO: 1.
[2] A method for measuring a concentration of renin in a sample using an antibody, wherein the antibody is the antibody described in [1].
[3] A kit for use in the method described in [2], comprising at least the antibody described in [1].
[4] composition for detecting renin in a sample, comprising the antibody described in [1].

### [Advantageous Effects of Invention]

The present invention makes it possible to obtain a measurement value of a renin concentration which is in correlation with renin activity even in the presence of a renin inhibitor in the same way as in the absence thereof. Thus, it is possible to accurately carry out a diagnosis even for a patient to whom a renin inhibitor has been administered. Additionally, the method of the present invention does not have such a problem that the measurement value is affected by the concentration of angiotensinogen in plasma, which is found in the method of measuring renin activity utilizing the index angiotensin-I being a degradation product of angiotensinogen by renin. Therefore, the present invention is extremely useful in e.g. the diagnosis of diseases associated with renin activity such as primary aldosteronism.

### [Brief Description of Drawings]

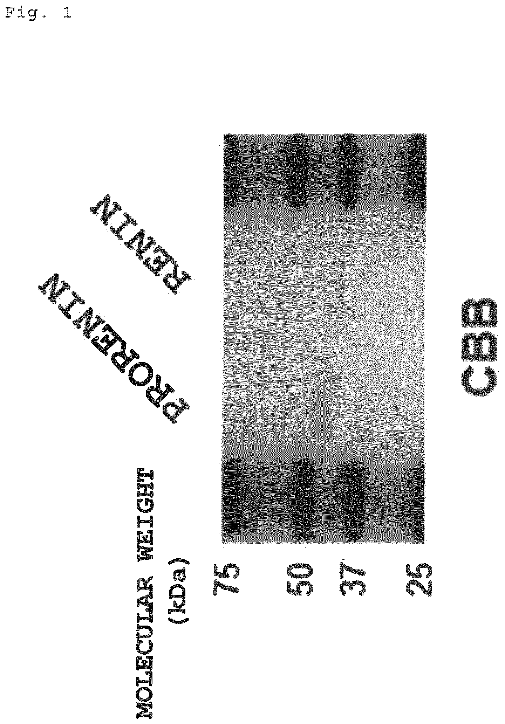

[Fig. 1] Fig. 1 is a photograph illustrating the results of detecting recombinant prorenin and recombinant mature renin by SDS-PAGE analysis.

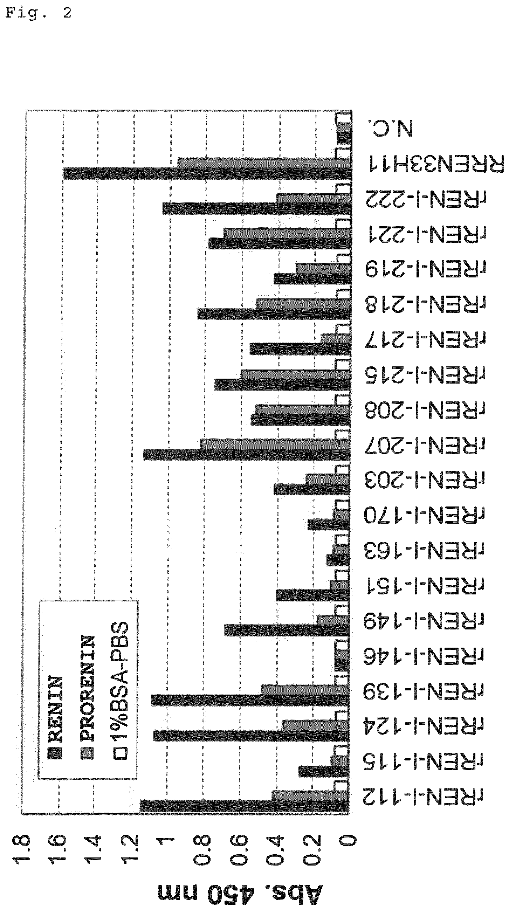

[Fig. 2] Fig. 2 is a graph illustrating the reactivity of the prepared anti-mature renin antibodies to mature renin and prorenin.

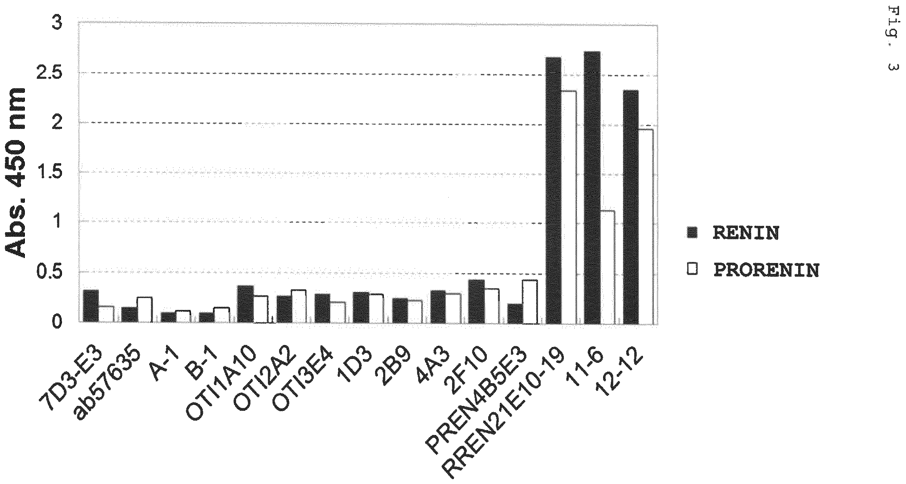

[Fig. 3] Fig. 3 is a graph illustrating the reactivity of the commercially available antibodies to mature renin and prorenin.

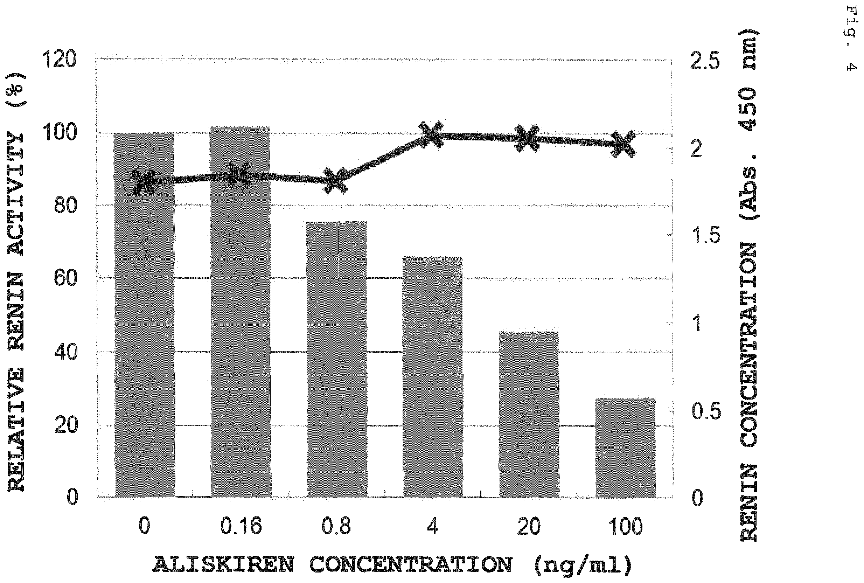

[Fig. 4] Fig. 4 is a graph illustrating the correlation between renin concentration and renin activity with the addition of aliskiren, obtained by sandwich ELISA using the combination of commercially available antibodies 12-12 and 11-6. The bar graphs indicate renin activity, and the line graph indicates renin concentration. The horizontal axis of the graph is taken as aliskiren concentration, and the vertical axes are taken as relative renin activity (left) and renin concentration (right).

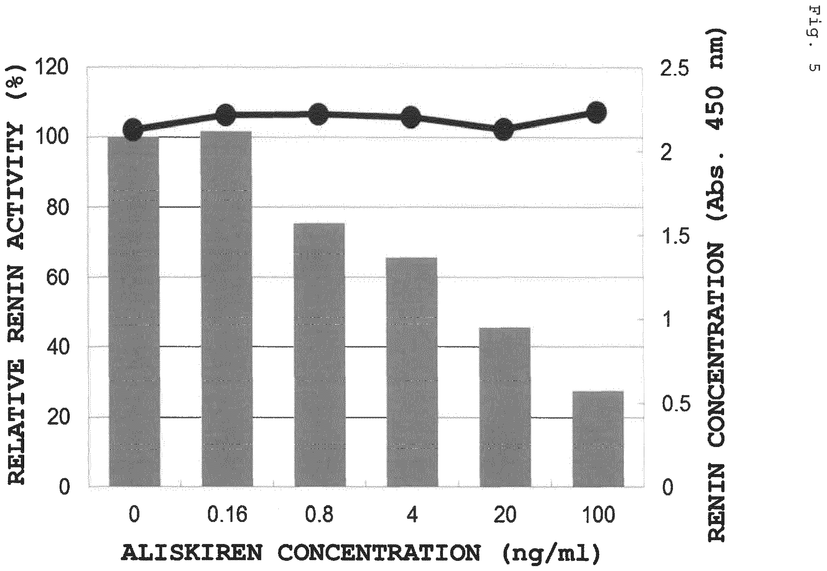

[Fig. 5] Fig. 5 is a graph illustrating the correlation between renin concentration and renin activity with the addition of aliskiren, obtained by sandwich ELISA using the combination of own company antibodies RREN33H11 and RREN21E10-19. The bar graphs indicate renin activity, and the line graph indicates renin concentration. The horizontal axis of the graph is taken as aliskiren concentration, and the vertical axes are taken as relative renin activity (left) and renin concentration (right).

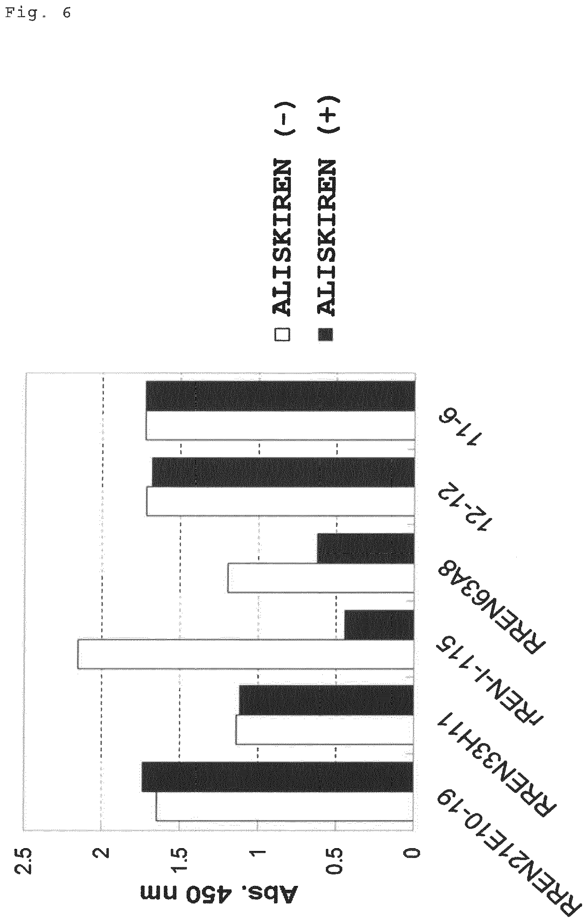

[Fig. 6] Fig. 6 is a graph illustrating reactivity of our company antibodies and commercially available antibodies to mature renin with the addition and without the addition of aliskiren.

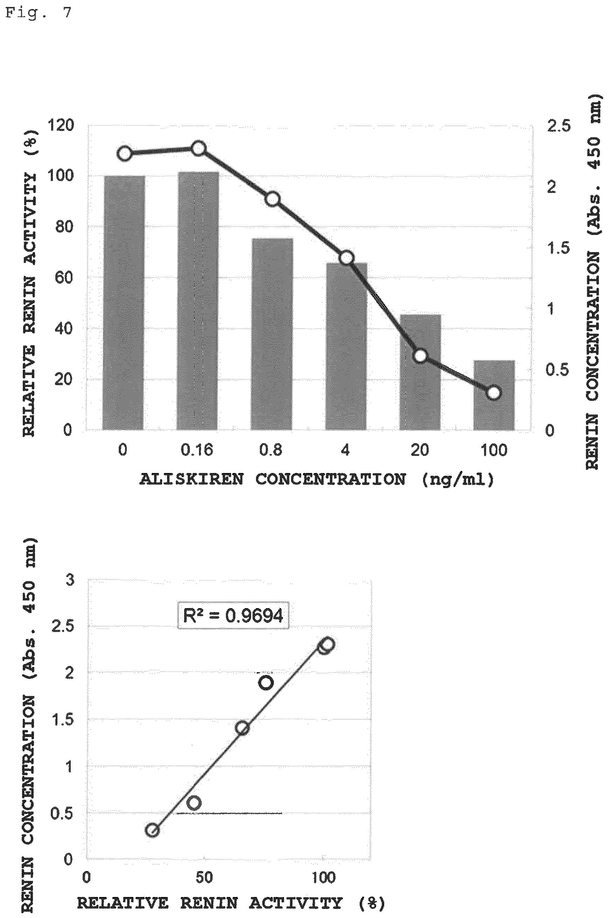

[Fig. 7] Fig. 7 provides graphs illustrating the correlation between renin concentration and renin activity with the addition of aliskiren, obtained by sandwich ELISA using the combination of our company antibodies rREN-I-115 and RREN21E10-19. For the upper figure, the bar graphs indicate renin activity, and the line graph indicates renin concentration. The horizontal axis of the graph is taken as aliskiren concentration, and the vertical axes are taken as relative renin activity (left) and renin concentration (right). For the lower figure, the horizontal axis of the graph is taken as relative renin activity, and the vertical axis is taken as renin concentration (Abs. 450 nm).

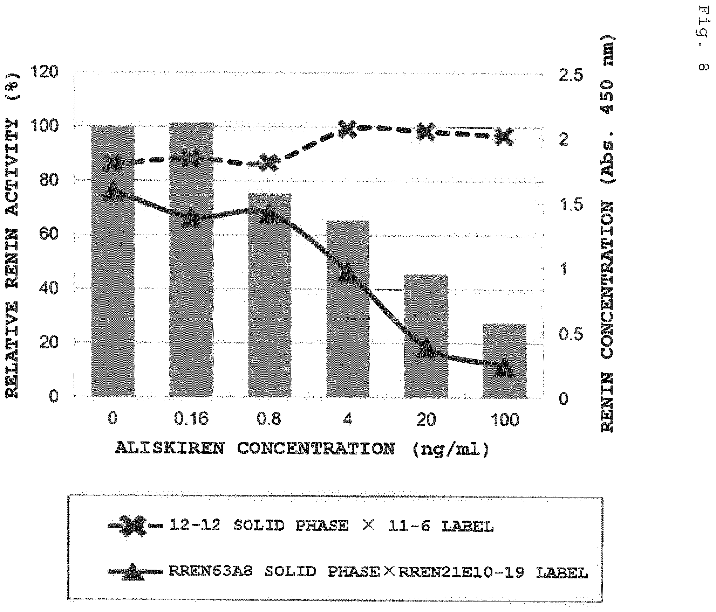

[Fig. 8] Fig. 8 is a graph illustrating the correlation between renin concentration and renin activity with the addition of aliskiren, obtained by sandwich ELISA using the combination of our company antibodies RREN63A8 and RREN21E10-19. The combination of commercially available antibodies 12-12 and 11-6 was used as a negative control. The bar graphs indicate renin activity, and the line graphs indicate renin concentration. The horizontal axis of the graph is taken as aliskiren concentration, and the vertical axes are taken as relative renin activity (left) and renin concentration (right).

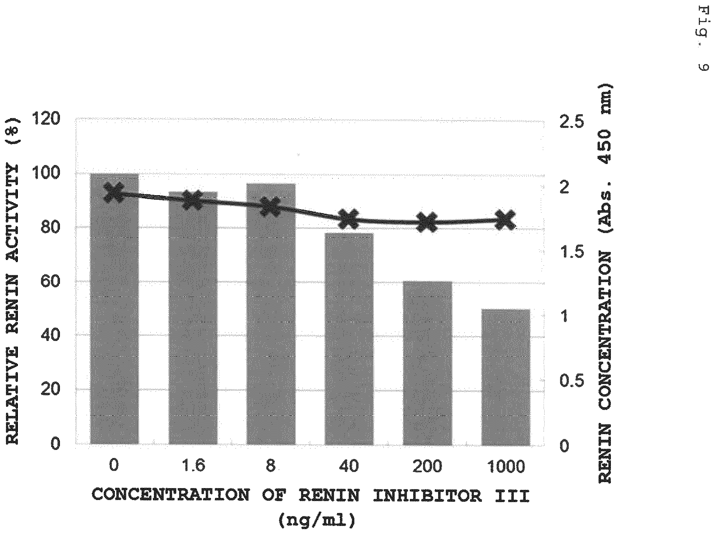

[Fig. 9] Fig. 9 is a graph illustrating the correlation between renin concentration and renin activity with the addition of Renin Inhibitor III, obtained by sandwich ELISA using the combination of commercially available antibodies 12-12 and 11-6. The bar graphs indicate renin activity, and the line graph indicates renin concentration. The horizontal axis of the graph is taken as aliskiren concentration, and the vertical axes are taken as relative renin activity (left) and renin concentration (right).

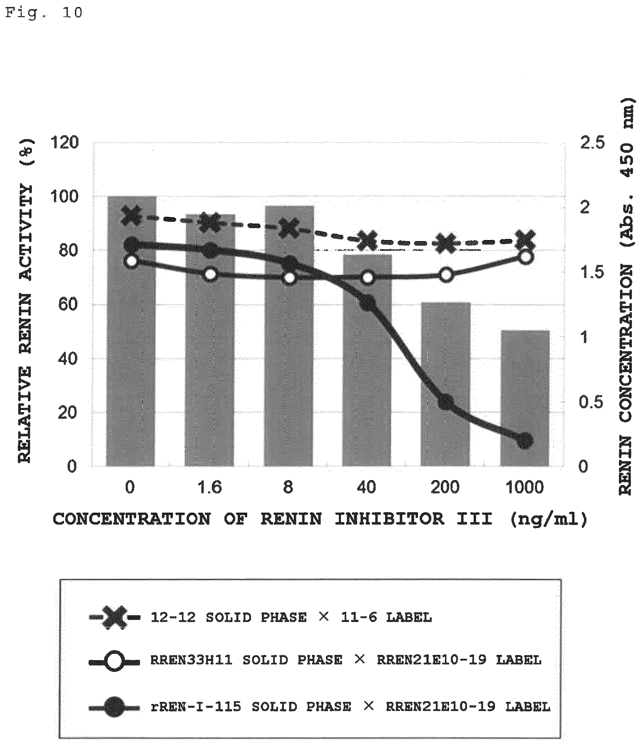

[Fig. 10] Fig. 10 is a graph illustrating the correlation between renin concentration and renin activity with the addition of Renin Inhibitor III, obtained by sandwich ELISA using RREN21E10-19 in combination with our company antibody rREN-I-115 or RREN33H11. The combination of commercially available antibodies 12-12 and 11-6 was used as a negative control. The bar graphs indicate renin activity, and the line graphs indicate renin concentration. The horizontal axis of the graph is taken as aliskiren concentration, and the vertical axes are taken as relative renin activity (left) and renin concentration (right).

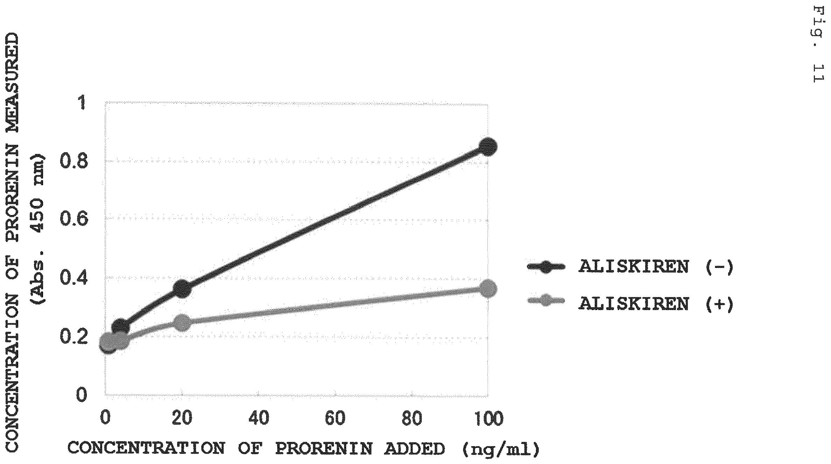

[Fig. 11] Fig. 11 is a graph illustrating the results of measuring the reactivity to prorenin with the addition and without the addition of aliskiren, obtained by sandwich ELISA using the combination of our company antibodies rREN-I-115 and RREN21E10-19. The horizontal axis of the graph is taken as the concentration of prorenin added, and the vertical axis is taken as the concentration of prorenin measured (Abs. 450 nm).

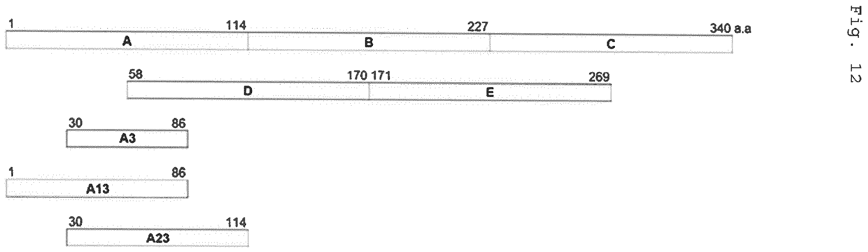

[Fig. 12] Fig. 12 is a graph illustrating renin fragments used for epitope analysis.

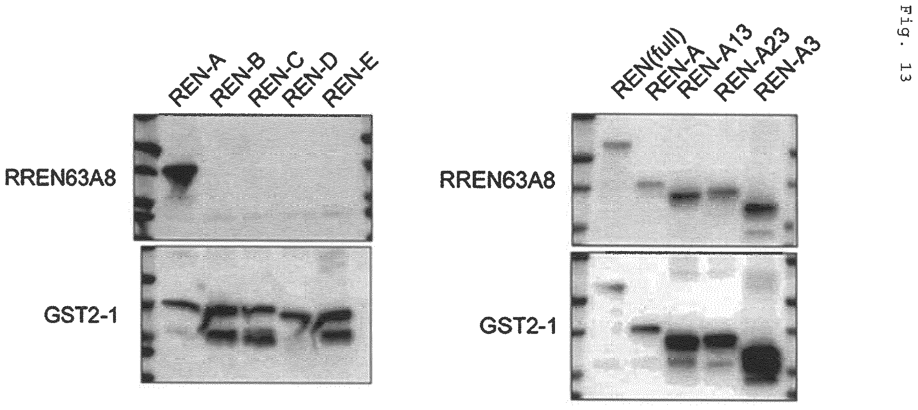

[Fig. 13] Fig. 13 provides photographs illustrating the results of identifying the epitope of our company antibody RREN63A8 by western blotting.

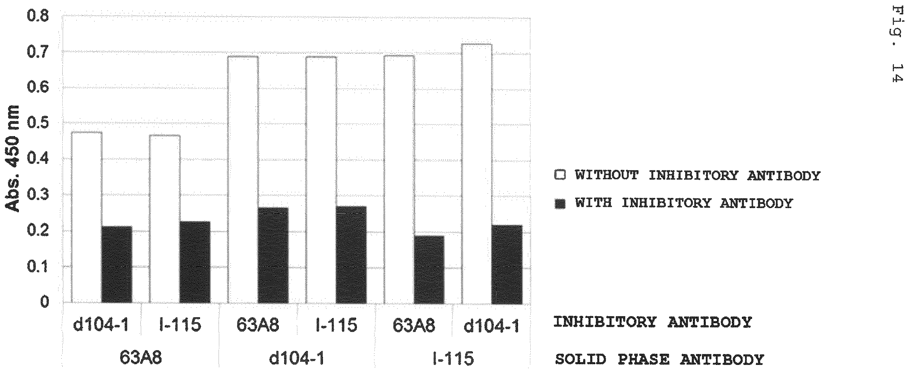

[Fig. 14] Fig. 14 is a graph illustrating the results of competitive inhibition experiments for our company antibodies (rREN-d-104-1, rREN-I-115, and RREN63A8).

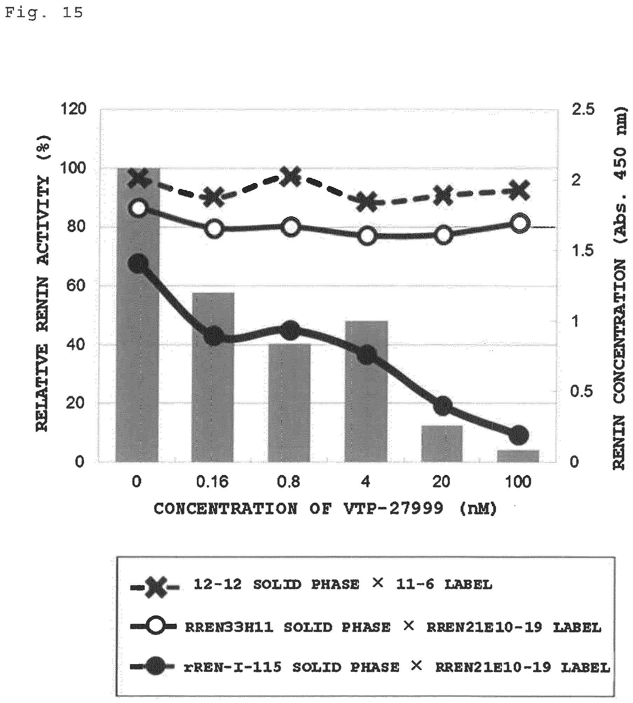

[Fig. 15] Fig. 15 is a graph illustrating the correlation between renin concentration and renin activity with the addition of VTP-27999, obtained by sandwich ELISA using RREN21E10-19 in combination with our company antibody rREN-I-115 or RREN33H11. The combination of commercially available antibodies 12-12 and 11-6 was used as a negative control. The bar graphs indicate renin activity, and the line graphs indicate renin concentration. The horizontal axis of the graph is taken as aliskiren concentration, and the vertical axes are taken as relative renin activity (left) and renin concentration (right).

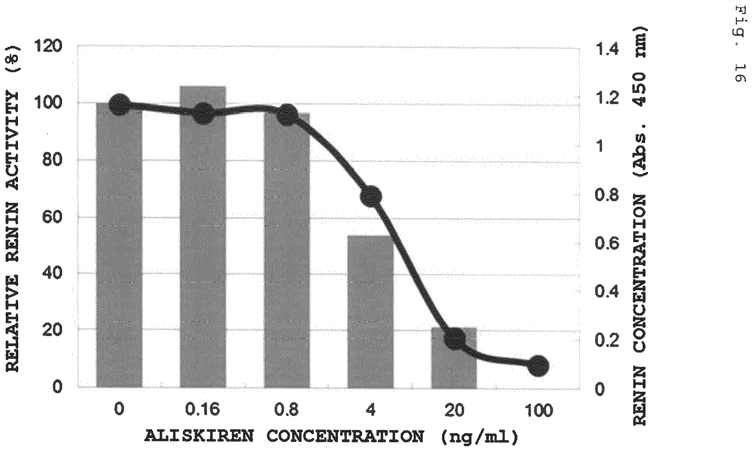

[Fig. 16] Fig. 16 is a graph illustrating the correlation between renin concentration and renin activity with the addition of aliskiren, obtained by sandwich ELISA using the combination of our company antibodies rREN-d-104-1 and RREN21E10-19. The bar graphs indicate renin activity, and the line graph indicates renin concentration. The horizontal axis of the graph is taken as aliskiren concentration, and the vertical axes are taken as relative renin activity (left) and renin concentration (right).

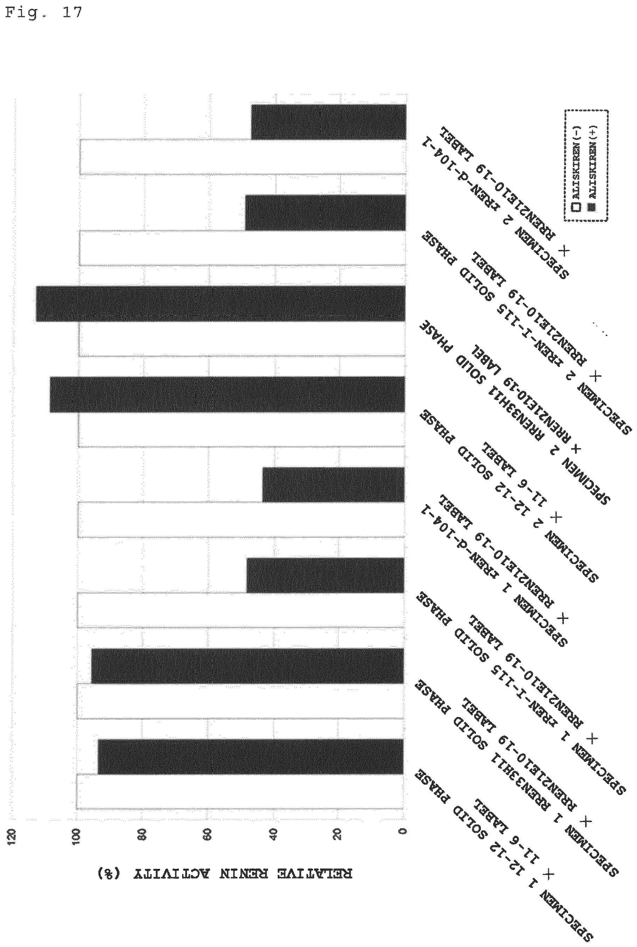

[Fig. 17] Fig. 17 is a graph illustrating the results of detecting reactivity to mature renin contained in human plasma with the addition and without the addition of aliskiren, obtained by sandwich ELISA using the combination of our company antibodies or the combination of commercially available antibodies.

### [Description of Embodiments]

The present invention provides an antibody which shows higher reactivity to active renin than to inactive renin (hereinafter referred to as the "active renin-specific antibody of the present invention"). Also, the present invention provides a method for measuring a concentration of renin in a sample using an antibody, wherein the antibody is the active renin-specific antibody of the present invention.

In the present invention, "renin" means mature renin and its precursor prorenin. Prorenin is classified into closed structure prorenin and open structure prorenin. Among these, mature renin and open structure prorenin originally have the activity of degrading angiotensinogen to produce angiotensin-I, and closed structure prorenin originally does not have this activity. However, when bound with a renin inhibitor, mature renin and open structure prorenin are inactivated. Therefore, in the present invention, "active renin" includes, among the above renins, mature renin and open structure prorenin not bound with a renin inhibitor, and "inactive renin" includes, among the above renins, closed structure prorenin not originally having activity as well as mature renin and open structure prorenin inactivated by binding of a renin inhibitor.

Examples of the "renin inhibitor" in the present invention include aliskiren, Renin Inhibitor III, and VTP-27999, but are not limited to these as long as they bind to renin and inhibit its activity. Note that aliskiren is commercially available from Novartis Pharmaceuticals as a drug for treating hypertension (trade name of Rasilez) . In addition, Renin Inhibitor III is a peptide having human renin inhibitory activity (Wood J. et al. Hypertension. 7, 797 (1985)) and is commercially available from KareBay Biochem. Moreover, VTP-27999 is an alkylamine compound having human renin inhibitory activity developed by Vitae Pharmaceuticals, Inc. (Lanqi Jia et al. ACS Med. Chem. Lett. 2, 747 (2011)) and is commercially available from Chemscene, LLC. It is possible to use these commercially available products in the evaluation of the reactivity of the active renin-specific antibody of the present invention to renin.

The active renin-specific antibody of the present invention is not particularly limited as long as it shows higher reactivity to active renin than to inactive renin. The phrase "shows higher reactivity to active renin than to inactive renin" means at least the showing of higher reactivity to active mature renin than to inactive mature renin. Preferably, it further means the showing of higher reactivity to active prorenin than to inactive prorenin.

Here, the "higher reactivity" is preferably reactivity of 1.5 times or more and more preferably 2 times or more (for example, 3 times or more, 4 times or more, or 5 times or more). The reactivity to inactive mature renin and active mature renin can be evaluated based on, for example, the absorbance value at 450 nm measured in accordance with the method described in Example 6 (mature renin concentration of 100 ng/ml). Additionally, the reactivity to inactive prorenin and active prorenin can be evaluated based on, for example, the absorbance value at 450 nm measured in accordance with the method described in Example 11 (prorenin concentration of 100 ng/ml).

The active renin-specific antibody of the present invention is a monoclonal antibody which binds to a peptide composed of 30th to 86th amino acids of an amino acid sequence set forth in SEQ ID NO: 1 (antibody having an epitope at the 30th to 86th amino acids of the amino acid sequence of human mature renin) . Moreover, the antibody is an antibody which does not bind to the peptide composed of the 58th to 170th amino acids of the amino acid sequence set forth in SEQ ID NO: 1. Specific examples of the antibody include "RREN63A8, " "rREN-I-115," and "rREN-d-104 -1" described in Examples. "RREN63A8" and "rREN-I-115" respectively show about twice and about five times the reactivity to mature renin not bound with a renin inhibitor compared with that to mature renin bound with a renin inhibitor (Fig. 6). Moreover, "rREN-I-115" shows about twice the reactivity to prorenin not bound with a renin inhibitor than to prorenin bound with a renin inhibitor (Fig. 11). The "rREN-d-104-1" shows the same specificity as that of "rREN-I-115" to active mature renin (Fig. 17). Thus, the renin concentration measured using these antibodies can strongly correlate with renin activity (Figs. 7, 8, 10, 15, and 16). In addition, other antibodies that recognize the same epitopes as those of "RREN63A8," "rREN-I-115," or "rREN-d-104-1" are also useful in the present invention because they are considered to have the same characteristics.

The "sample" used in the measurement of the present invention is not particularly limited as long as it is a sample in which renin can exist. Blood specimen is generally used for the purpose of measuring renin concentration, which serves as the basis of diagnosis of diseases associated with abnormality of renin activity. The blood specimen is preferably serum or plasma.

Examples of the method for immunologically measuring a renin concentration using the active renin-specific antibody of the present invention include, but not limited to, the RIA method (radioimmunoassay) using a radioactive isotope as a label, the CLIA method (chemiluminescent immunoassay) using a chemiluminescent compound as a label, the CLEIA method (chemiluminescent enzyme immunoassay) as well as the EIA method (enzyme immunoassay) using an enzyme as a label, the latex agglutination method, and immunochromatography. The immunological measurement may be a noncompetitive measurement method or a competitive measurement method.

The antibodies used in the immunological method may be of any isotype such as IgG, IgM, IgA, IgD, IgE, and IgY. These antibodies may also be polyclonal antibodies or monoclonal antibodies (for example, chimeric antibodies, humanized antibodies, and human antibodies), or antibody fragments thereof. Examples of the antibody fragments include, but not limited to, F(ab')2, Fab', Fab, Fv, single-chain antibodies, and diabodies. The antibodies are preferably monoclonal antibodies. It is possible to use, as the monoclonal antibodies, those prepared by a known method such as the hybridoma method or the recombinant DNA method.

As described in Examples, the preparation of the monoclonal antibodies used in the present invention includes first preparing monoclonal antibodies against mature renin and selecting the monoclonal antibodies having high reactivity to mature renin but low reactivity to prorenin. Next, from the selected monoclonal antibodies, one may select the antibodies whose measurement value correlates with renin activity regardless of the presence or absence of a renin inhibitor. In addition, once the active renin-specific antibody of the present invention is obtained, identification of its epitope and use of a peptide containing the epitope make it possible to efficiently obtain the target active renin-specific antibody.

Representative examples of the hybridoma method include the method by Kohler and Milstein (Kohler & Milstein, Nature, 256: 495 (1975)). The antibody-producing cells used in the cell fusion step in this method are, for example, spleen cells, lymph node cells, and peripheral blood leukocytes of an animal (for example, mouse, rat, hamster, rabbit, monkey, goat, sheep, donkey, camel, alpaca, or chicken) immunized with an antigen (such as the target protein, a partial peptide thereof, or a cell expressing them) . It is also possible to use antibody-producing cells obtained by allowing an antigen to act in a medium on the above-described cells or lymphocytes isolated in advance from an unimmunized animal. It is possible to use various known cell lines as myeloma cells. The antibody-producing cells and myeloma cells may be of different animal species origins as long as they are capable of fusion, but are preferably of the same animal species origin. Hybridomas are produced by, for example, cell fusion between spleen cells obtained from a mouse immunized with an antigen and mouse myeloma cells, and the subsequent screening makes it possible to obtain hybridomas which produce monoclonal antibodies specific to the target protein. The monoclonal antibodies against the target protein can be acquired by culturing hybridomas or from the ascites of a mammal to which the hybridomas have been administered.

The recombinant DNA method is a method in which a DNA encoding the above antibodies is cloned from e.g. a hybridoma or a B cell and incorporated into an appropriate vector, followed by introduction into a host cell (such as a mammalian cell line, E. coli, a yeast cell, an insect cell, or a plant cell) to allow production as a recombinant antibody (for example, P. J. Delves, Antibody Production: Essential Techniques, 1997 WILEY, P. Shepherd and C. Dean Monoclonal Antibodies,2000 OXFORD UNIVERSITY PRESS, Vandamme A. M. et al., Eur. J. Biochem. 192: 767-775 (1990)). In the expression of a DNA encoding an antibody, DNAs encoding heavy chain or light chain may be separately incorporated into expression vectors to transform the host cell, or a DNA encoding heavy and light chains may be incorporated into a single expression vector to transform the host cell (see International Publication No. WO94/11523). A recombinant antibody can be acquired in a substantially pure and homogeneous form by culturing the above host cell, followed by isolation and purification in the host cell or the culture solution. In the isolation and purification of the antibody, it is possible to use a method used for ordinary purification of a polypeptide. If a transgenic animal production technique is used to prepare a transgenic animal (such as a cow, a goat, a sheep, or a pig) having an antibody gene incorporated therein, it is also possible to acquire, from the milk of that transgenic animal, monoclonal antibodies in a large amount derived from the antibody gene.

It is possible to use an antibody bound with a labeling substance as the active renin-specific antibody used in the measurement of the present invention. Although the labeling substance is not particularly limited as long as it can be detected when bound with an antibody, examples thereof include radioactive isotopes such as 125I, enzymes such as alkaline phosphatase (ALP), horseradish peroxidase (HRP), and β-galactosidase (β-gal), fluorescent dyes such as fluorescein isothiocyanate (FITC) and rhodamine isothiocyanate (RITC), fluorescent proteins such as allophycocyanin (APC) and phycoerythrin (R-PE), avidin, biotin, latex, and gold particles.

In the case of using an enzyme as the labeling substance, it is possible to carry out various detections depending on the substrate by adding, as the substrate, a chromogenic substrate, a fluorescent substrate, a chemiluminescent substrate, or the like.

In addition to the method for directly detecting renin using an active renin-specific antibody bound with a labeling substance, it is possible to use an indirect detection method using e.g. a secondary antibody bound with a labeling substance instead of using the active renin-specific antibody of the present invention bound with a labeling substance. Here, the "secondary antibody" is an antibody which shows reactivity to the active renin-specific antibody of the present invention. When the active renin-specific antibody of the present invention is prepared as, for example, a mouse antibody, it is possible to use an anti-mouse IgG antibody as the secondary antibody. Labeled secondary antibodies usable for antibodies derived from various species of organisms such as rabbits, goats, and mice are commercially available, and it is possible to select and use an appropriate secondary antibody depending on the organism from which the active renin-specific antibody of the present invention is derived. It is also possible to use e.g. protein G or protein A bound with a labeling substance instead of the secondary antibody.

A biotin-avidin system can also be used for the binding between the active renin-specific antibody of the present invention and a labeling substance. For example, in this method, the active renin-specific antibody of the present invention is biotinylated and acted upon by an avidinated labeling substance, followed by use of the interaction between biotin and avidin to bind the labeling substance to the active renin-specific antibody of the present invention.

The detection principle of the immunological measurement method used in the present invention is preferably the sandwich method from the viewpoint that it is possible to construct a highly sensitive detection system. In the sandwich method, the substance to be detected is captured with an immobilized capture antibody and recognized by a detection antibody bound with a labeling substance, followed by washing to carry out detection depending on the type of the labeling substance. Examples of usable solid phases include particles such as magnetic particles and latex particles, plates such as plastic plates, or fibrous substances such as nitrocellulose.

The capture antibody may be directly immobilized or indirectly immobilized on the solid phase. It is possible to indirectly immobilize the capture antibody on the solid phase by, for example, immobilizing a substance which binds to the capture antibody on the solid phase and binding the capture antibody to the substance. Examples of the substance which binds to the capture antibody include, but not limited to, the secondary antibody, protein G, and protein A described above. In addition, if the capture antibody is biotinylated, an avidinated solid phase can be used.

In the present invention, the active renin-specific antibody may be used as the capture antibody or may be used as the detection antibody (labeled antibody). Thus, in the case of using the sandwich method, it is not always necessary to label the active renin-specific antibody of the present invention, and the other antibody may be labeled. The other antibody may be one that shows reactivity to mature renin bound with a renin inhibitor as long as it shows reactivity at least to mature renin not bound with a renin inhibitor. Moreover, reactivity may be shown to prorenin. It is possible to preferably use, for example, antibodies which show high reactivity to renin in general regardless of the presence or absence of a renin inhibitor (such as RREN21E10-19 of Examples) and antibodies which do not show high reactivity to prorenin but show high reactivity to mature renin regardless of the presence or absence of a renin inhibitor (such as 11-6 of Examples) (Figs. 3 and 6). Preferable examples of the sandwich method include the sandwich CLIA method which is a form of the CLIA method, the sandwich CLEIA method which is a form of the CLEIA method, and the IRMA method (immunoradiometric assay) which is a form of the RIA method.

In general, the renin concentration can be determined from the obtained measurement value by comparison with the measured values using standard renin reagents of various concentrations. In this case, it is possible to obtain the concentration of renin in the sample by checking, for example, the position of the actual measurement value on the standard curve created based on measurement values by the standard renin reagents.

The concentration of renin in the sample measured by the present invention correlates with the renin activity in the sample. Thus, it is possible to use the method of the present invention for the diagnosis of diseases associated with abnormality of renin activity (morbidity and evaluation of its risk). Here, the "diseases associated with abnormality of renin activity" are meant to include both diseases caused by abnormality of renin activity and diseases whose onset results in abnormality of renin activity. In the diseases associated with abnormality of renin activity, examples of the diseases having a high value of renin activity include malignant hypertension, pheochromocytoma, primary selective hypoaldosteronism, renovascular hypertension, renal parenchymal hypertension, chronic heart failure, nephrotic syndrome, cirrhosis of the liver, dehydration, Addison's disease, Bartter syndrome, Gitelman's syndrome, renin-producing tumor, and high renin essential hypertension, and examples of the diseases having a low value of renin activity include primary aldosteronism, low renin essential hypertension, interstitial nephritis, diabetic nephropathy, pyelonephritis, 11β-hydroxylase deficiency, 17α-hydroxylase deficiency, AME syndrome, DOC-producing tumor, Liddle's syndrome, pseudoaldosteronism, congenital adrenal enzyme deficiency, low-renin selective hypoaldosteronism, glucocorticoid remediable aldosteronism, and idiopathic aldosteronism.

In addition, the present invention provides a composition for detecting renin in a sample, comprising the active renin-specific antibody of the present invention. The active renin-specific antibody contained in the drug of the present invention may be one bound with a labeling substance as described above. In addition to antibody components, the drug of the present invention can contain, when necessary, other components such as sterilized water, physiological saline, buffer, and preservative.

Also, the present invention provides a kit for use in the above method of the present invention. The kit of the present invention contains at least the active renin-specific antibody of the present invention as an antibody preparation, and can be combined further with a standard renin reagent (various concentrations), a control reagent, a diluted solution of the sample, a diluting cartridge, a wash solution, and the like. In the case of using an enzyme label, it is possible to contain a substrate and a reaction terminating solution necessary for detecting a label. In the case of using the sandwich method as the detection principle, it is possible to contain, for example, an immobilized renin capture antibody or a solid phase immobilized with a substance that binds to renin capture antibody. In addition, in the case of not labeling a primary antibody, for example, one labeled with a substance that binds to the primary antibody can be contained in the kit. Moreover, when the active renin-specific antibody of the present invention is biotinylated, an avidinated labeling substance can be contained in the kit, for example. The kit of the present invention can further contain an instruction manual for use of the kit.

The drug and the kit of the present invention can be used as, for example, an in-vitro diagnostic medicine in the measurement of the renin concentration which serves as the basis of diagnosis of diseases associated with abnormality of renin activity.

### [Examples]

Hereinafter, the present invention is described more specifically based on Examples, but the present invention is not limited to Examples below.

### [Example 1] Preparation of Recombinant Mature Renin

### (1) Purification of Recombinant Prorenin

A high-density culture unit AD1000 was used to culture His-tag-label prorenin-expressing CHO cells for 1 week in a serum-free medium BD select (BD Biosciences) . The culture supernatant was recovered and centrifuged at 4°C and 1000 rpm for 10 minutes, followed by recovery of the supernatant. The supernatant was passed through an affinity column for His tag purification (Talon Metal Affinity Resin (Clontech)). After washing with phosphate buffered saline (PBS), elution was carried out with a 150 mM imidazole solution, followed by recovery of 10 fractions of 1 ml each of the eluate. SDS-PAGE analysis was carried out on the recovered fractions, and after the elution of prorenin was confirmed, all fractions were collected to carry out dialysis overnight in PBS. On the next day, the dialyzed solution was recovered and concentrated with an ultrafiltration column (Amicon Ultra 10K) to recover the prorenin solution.

### (2) Purification of Recombinant Mature Renin

To the recombinant prorenin purified in (1), a trypsin (Sequencing grade modified Trypsin, Promega) solution (0.1 mg/mL, 50 mM Acetate) was added so that the weight ratio was 1: 50 and the mixture was reacted at 4°C for 1 hour, followed by centrifugation at 15000rpm, RT for 5 minutes to recover the supernatant. The recovered supernatant was purified by gel filtration chromatography (column: Superdex 75 10/300) with a 20 mM Tris-HCl (pH 7.0) solution. Fraction analysis was carried out by SDS-PAGE to recover the fractions containing mature renin. The recovered solution was dialyzed overnight in PBS. On the next day, the dialyzed solution was recovered and concentrated with an ultrafiltration column (Amicon Ultra 10K) .

### (3) SDS-PAGE analysis of Recombinant Prorenin and Recombinant Mature Renin

To a solution of recombinant prorenin and a solution of recombinant mature renin adjusted to 20 µg/mL, 2 × SDS-PAGE sample buffer (2 × Laemmli sample buffer, BioRad) containing an equal amount of 100 mM DTT was added, followed by denaturing at 95°C for 5 minutes . The prepared sample was added to a precast gel for SDS-PAGE (Mini-PROTEAN PGX Precast Gel 4-20%) at 10 µL/lane. The gel was set in an electrophoresis apparatus and electrophoresed at a constant voltage of 200 V for 24 minutes. After the gel was washed with ultrapure water, GelCode Blue Safe Proteinstain (Thermo Scientific) was added, followed by staining at room temperature for about 2 hours. The stained gel was decolorized with ultrapure water and photographed. As a result, it was confirmed that recombinant prorenin and recombinant mature renin had been purified (Fig. 1).

### [Example 2] Acquisition of Mature Renin-Specific Antibody

### (1) Mouse Immunity

A BALB/c mouse and an ICR mouse (female) of 7 to 8 weeks of age were injected intraperitoneally with 50 µg/body of recombinant mature renin and an equivalent amount of emulsion of Freund's complete adjuvant. Thereafter, the mice were intraperitoneally injected every 2 weeks with 50 µg/body of recombinant mature renin and an equivalent amount of emulsion of incomplete Freund's adjuvant 2 to 5 times until an increase in antibody titer was observed.

### (2) Preparation of Anti-Mature Renin Antibody-Producing Hybridomas

The mice for which a sufficient increase in antibody titer was observed were intraperitoneally injected with 50 µg/body of the solution of recombinant mature renin. After 3 to 4 days, the mouse spleen was excised, and spleen cells as well as PEG and myeloma cells P3U1 previously cultured in RPMI 1640 were used for fusion. The culture supernatant 7 to 14 days after the fusion was added to a washed plate immobilized with an anti-mouse immunoglobulin antibody, followed by a primary reaction. After washing three times with PBS-T, a solution of biotinylated prorenin or a solution of recombinant renin (1 µg/mL) biotinylated with a commercially available biotinylating reagent (Sulfo-NHS-LC biotin, Thermo Scientific) was added to carry out a secondary reaction. After washing three times with PBS-T, a solution of POD-labeled streptavidin was added to carry out a tertiary reaction. After washing three times with PBS-T, ABTS or TMB was added to carry out a chromogenic reaction. A 1.5 M aqueous solution of oxalic acid or 0.5 M H2SO4 was added to stop the chromogenic reaction, and the absorbance at 405 nm or 450 nm was measured. Clones highly reactive to renin were selected, and cloning by limiting dilution was undergone to establish monoclonal antibody-producing hybridomas.

### (3) Purification of Anti-Mature Renin Antibody

The hybridomas were cultured in a serum-free medium for about 1 to 2 weeks, and their culture supernatant was recovered. The culture supernatant was passed through a Protein G column and bound with an antibody. After washing with PBS, the antibody was eluted with an eluate (0.2 M Glycine-HCl, pH 2.5), followed by neutralization with the addition of a neutralizing solution (1 M Tris-HCl, pH 9.0). The antibody solution was dialyzed overnight with PBS and concentrated with an ultrafiltration column.

### (4) Study of Reactivity to Biotinylated Mature Renin and Biotinylated Prorenin

To an assay plate, 2 µg/mL of a solution of anti-mouse immunoglobulin antibody diluted with PBS was added, followed by immobilization. After washing three times with PBS-T, 1% BSA-PBS was added to the assay plate to carry out blocking. After washing three times with PBS-T, a solution of anti-mature renin antibody diluted with 1% BSA-PBS was added to carry out a primary reaction. After washing three times with PBS-T, 1 µg/mL of biotinylated renin and biotinylated prorenin diluted with 1% BSA-PBS was added to carry out a secondary reaction. After washing three times with PBS-T, a solution of POD-labeled streptavidin diluted with 1% BSA-PBS was added to carry out a tertiary reaction. After washing three times with PBS-T, TMB was added to carry out a chromogenic reaction. The 0.5 M H2SO4 was added to stop the chromogenic reaction, and the absorbance at 405 nm was measured. As a result, some clones showed high specificity to biotinylated mature renin (Fig. 2).

### [Example 3] Study of Reactivity of Commercially Available Antibody

To an assay plate, 2 µg/mL of a solution of anti-mouse immunoglobulin antibody diluted with PBS was added, followed by immobilization. After washing three times with PBS-T, 1% BSA-PBE was added to the assay plate to carry out blocking. After washing three times with PBS-T, 1 ug/mL each of the commercially available antibodies and our company antibodies diluted with 1% BSA-PBS was added to carry out a primary reaction. After washing three times with PBS-T, 1 µg/mL of biotinylated renin and biotinylated prorenin diluted with 1% BSA-PBS was added to carry out a secondary reaction. After washing three times with PBS-T, a solution of POD-labeled streptavidin diluted with 1% BSA-PBS was added to carry out a tertiary reaction. After washing three times with PBS-T, TMB was added to carry out a chromogenic reaction. The 0.5 M H2SO4 was added to stop the chromogenic reaction, and the absorbance at 405 nm was measured. As a result, commercially available antibody 11-6 showed high specificity to mature renin (Fig. 3).

### [Example 4] Measurement of Renin Concentration with Addition of Aliskiren by Sandwich ELISA Using Combination of Commercially Available Antibodies

### (1) Measurement of Renin Concentration

To an assay plate, 2 µg/mL of commercially available antibody 12-12 diluted with PBS was added, followed by immobilization. Subsequent to washing three times with PBS-T, 1% BSA-PBE was added to the assay plate to carry out blocking. Subsequent to washing three times with PBS-T, a mixture in equal amounts of 1 µg/ml of a solution of recombinant mature renin diluted with PBS and each of 200, 40, 8, 1.6, and 0.32 ng/mL of aliskiren solution was added to the assay plate, followed by a primary reaction. Subsequent to washing three times with PBS-T, biotinylated labeled commercially available antibody 11-6 diluted with 1% BSA-PBS was added to the assay plate, followed by a secondary reaction. Subsequent to washing three times with PBS-T, a solution of POD-labeled streptavidin diluted with 1% BSA-PBS was added to carry out a tertiary reaction. Subsequent to washing three times with PBS-T, TMB was added to carry out a chromogenic reaction. The 0.5 M H2SO4 was added to stop the chromogenic reaction, and the absorbance at 405 nm was measured.

### (2) Measurement of Renin Activity

To a white plate, 160 µL of assay buffer (50 mM Tris, 0.1 M NaCl, 10 mM EDTA) was added. Next, 20 µL of 20 µM renin fluorescent substrate (DMSO, Cayman Chemical) was added, and 20 µL of a mixture in equal amounts of a solution of recombinant mature renin and a solution of aliskiren (same sample as that used in the concentration measurement) and a renin concentration calibrator were further added. Reaction was carried out at 37°C for 1 hour to detect fluorescence at 490 nm (excitation light: 340 nm) .

As a result, sandwich ELISA using the combination of commercially available antibodies 12-12 and 11-6 with the addition of aliskiren showed no correlation between renin concentration and renin activity (Fig. 4).

### [Example 5] Measurement of Renin Concentration with Addition of Aliskiren by Sandwich ELISA Using Combination of Our Company Antibodies RREN33H11 and RREN21E10-19

### (1) Measurement of Renin Concentration

To an assay plate, 2 µg/mL of a solution of our company antibody RREN33H11 diluted with PBS was added, followed by immobilization. Subsequent to washing three times with PBS-T, 1% BSA-PBS was added to carry out blocking. Subsequent to washing three times with PBS-T, a mixture in equal amounts of 1 µg/ml of a solution of recombinant mature renin diluted with PBS and each of 200, 40, 8, 1.6, and 0.32 ng/mL of aliskiren solution was added to the assay plate, followed by a primary reaction. Subsequent to washing three times with PBS-T, biotinylated labeled RREN21E10-19 diluted with 1% BSA-PBS was added to the assay plate, followed by a secondary reaction. Subsequent to washing three times with PBS-T, a solution of POD-labeled streptavidin diluted with 1% BSA-PBS was added to carry out a tertiary reaction. Subsequent to washing three times with PBS-T, TMB was added to carry out a chromogenic reaction. The 0.5 M H2SO4 was added to stop the chromogenic reaction, and the absorbance at 405 nm was measured.

### (2) Measurement of Renin Activity

To a white plate, 160 µL of assay buffer (50 mM Tris, 0.1 M NaCl, 10 mM EDTA) was added. Next, 20 µL of 20 µM renin fluorescent substrate (DMSO, Cayman Chemical) was added, and 20 µL of a mixture in equal amounts of a solution of recombinant mature renin and a solution of aliskiren (same sample as that used in the concentration measurement) and a renin concentration calibrator were further added. Reaction was carried out at 37°C for 1 hour to detect fluorescence at 490 nm (excitation light: 340 nm) .

As a result, even in sandwich ELISA using the combination of our own antibodies RREN33H11 and RREN21E10-19 established with specificity to mature renin as an indicator, no correlation was observed between renin concentration and renin activity with the addition of aliskiren (Fig. 5).

### [Example 6] Establishment of Screening Method Using Biotinylated Renin Capturing Ability with Addition of Aliskiren and Acquisition of Active Renin-Specific Antibody

### (1) Measurement of Biotinylated Renin Capturing Ability with Addition of Aliskiren

To an assay plate, 2 µg/mL of anti-renin antibody solution diluted with PBS was added, followed by immobilization. Subsequent to washing three times with PBS-T, 1% BSA-PBS was added to carry out blocking. Subsequent to washing three times with PBS-T, a mixture in equal amounts of 1 ug/ml of a solution of biotinylated mature renin diluted with PBS and 200 ng/mL of aliskiren solution was added, followed by a primary reaction. Subsequent to washing three times with PBS-T, a solution of POD-labeled streptavidin diluted with 1% BSA-PBS was added to carry out a secondary reaction. Subsequent to washing three times with PBS-T, TMB was added to carry out a chromogenic reaction. The 0.5 M H2SO4 was added to stop the chromogenic reaction, and the absorbance at 405 nm was measured.

As a result, by measuring the biotinylated mature renin capturing ability with the addition of aliskiren, it was possible to construct a screening system with the reactivity to mature renin in the presence of aliskiren as an index. In addition, clones were obtained in which reactivity to mature renin markedly decreased with the addition of aliskiren (Fig. 6).

### [Example 7] Measurement of Renin Concentration with Addition of Aliskiren by Sandwich ELISA Using Combination of Our Company Antibodies rREN-I-115 and RREN21E10-19

### (1) Measurement of Renin Concentration

To an assay plate, 2 ug/mL of a solution of our company antibody rREN-I-115 diluted with PBS was added, followed by immobilization. Subsequent to washing three times with PBS-T, 1% BSA-PBS was added to carry out blocking. Subsequent to washing three times with PBS-T, a mixture in equal amounts of 1 µg/ml of a solution of recombinant mature renin diluted with PBS and each of 200, 40, 8, 1.6, and 0 .32 ng/mL of aliskiren solution was added to the assay plate, followed by a primary reaction. Subsequent to washing three times with PBS-T, biotinylated labeled RREN21E10-19 diluted with 1% BSA-PBS was added to the assay plate, followed by a secondary reaction. Subsequent to washing three times with PBS-T, a solution of POD-labeled streptavidin diluted with 1% BSA-PBS was added to carry out a tertiary reaction. Subsequent to washing three times with PBS-T, TMB was added to carry out a chromogenic reaction. The 0.5 M H2SO4 was added to stop the chromogenic reaction, and the absorbance at 405 nm was measured.

### (2) Measurement of Renin Activity

To a white plate, 160 µL of assay buffer (50 mM Tris, 0.1 M NaCl, 10 mM EDTA) was added. Next, 20 µL of 20 µM renin fluorescent substrate (DMSO, Cayman Chemical) was added, and 20 µL of a mixture in equal amounts of a solution of recombinant mature renin and a solution of aliskiren (same sample as that used in the concentration measurement) and a renin concentration calibrator were further added. Reaction was carried out at 37°C for 1 hour to detect fluorescence at 490 nm (excitation light: 340 nm) .

As a result, sandwich ELISA using the combination of our company antibodies rREN-I-115 and RREN21E10-19 selected with the reactivity to mature renin in the presence of aliskiren as an index showed strong correlation between renin concentration and renin activity (Fig. 7).

### [Example 8] Measurement of Renin Concentration with Addition of Aliskiren by Sandwich ELISA Using Combination of Our Company Antibodies RREN63A8 and RREN21E10-19

### (1) Measurement of Renin Concentration

To an assay plate, 2 ug/mL of a solution of our company antibody RREN63A8 diluted with PBS was added, followed by immobilization. Subsequent to washing three times with PBS-T, 1% BSA-PBS was added to carry out blocking. Subsequent to washing three times with PBS-T, a mixture in equal amounts of 1 µg/ml of a solution of recombinant mature renin diluted with PBS and each of 200, 40, 8, 1.6, and 0.32 ng/mL of aliskiren solution was added to the assay plate, followed by a primary reaction. Subsequent to washing three times with PBS-T, biotinylated labeled RREN21E10-19 diluted with 1 % BSA-PBS was added to the assay plate, followed by a secondary reaction. Subsequent to washing three times with PBS-T, a solution of POD-labeled streptavidin diluted with 1% BSA-PBS was added to carry out a tertiary reaction. Subsequent to washing three times with PBS-T, TMB was added to carry out a chromogenic reaction. The 0.5 M H2SO4 was added to stop the chromogenic reaction, and the absorbance at 405 nm was measured.

### (2) Measurement of Renin Activity

To a white plate, 160 µL of assay buffer (50 mM Tris, 0.1 M NaCl, 10 mM EDTA) was added. Next, 20 µL of 20 µM renin fluorescent substrate (DMSO, Cayman Chemical) was added, and 20 µL of a mixture in equal amounts of a solution of recombinant mature renin and a solution of aliskiren (same sample as that used in the concentration measurement) and a renin concentration calibrator were further added. Reaction was carried out at 37°C for 1 hour to detect fluorescence at 490 nm (excitation light: 340 nm) .

As a result, as in the case of rREN-I-115, sandwich ELISA using the combination with clone RREN63A8 selected with the reactivity to mature renin in the presence of aliskiren as an index showed correlation between renin concentration and renin activity (Fig. 8).

### [Example 9] Measurement of Renin Concentration with Addition of Renin Inhibitor III by Sandwich ELISA Using Combination of Commercially Available Antibodies 12-12 and 11-6

### (1) Measurement of Renin Concentration

To an assay plate, 2 ug/mL of commercially available antibody 12-12 diluted with PBS was added, followed by immobilization. Subsequent to washing three times with PBS-T, 1% BSA-PBE was added to the assay plate to carry out blocking. Subsequent to washing three times with PBS-T, a mixture in equal amounts of 200 ng/ml of a solution of recombinant mature renin diluted with PBS and each of 2000, 400, 80, 16, and 3.2 ng/mL of a solution of Renin Inhibitor III was added to the assay plate, followed by a primary reaction. Subsequent to washing three times with PBS-T, biotinylated labeled commercially available antibody 11-6 diluted with 1% BSA-PBS was added to the assay plate, followed by a secondary reaction. Subsequent to washing three times with PBS-T, a solution of POD-labeled streptavidin diluted with 1% BSA-PBS was added to carry out a tertiary reaction. Subsequent to washing three times with PBS-T, TMB was added to carry out a chromogenic reaction. The 0.5 M H2SO4 was added to stop the chromogenic reaction, and the absorbance at 405 nm was measured.

### (2) Measurement of Renin Activity

To a white plate, 160 µL of assay buffer (50 mM Tris, 0.1 M NaCl, 10 mM EDTA) was added. Next, 20 µL of 20 µM renin fluorescent substrate (DMSO, Cayman Chemical) was added, and 20 µL of a mixture in equal amounts of a solution of recombinant mature renin and a solution of Renin Inhibitor III (same sample as that used in the concentration measurement) and a renin concentration calibrator were further added. Reaction was carried out at 37°C for 1 hour to detect fluorescence at 490 nm (excitation light: 340 nm).

As a result, the combination of commercially available antibodies showed no correlation between renin concentration and renin activity even with the addition of a renin inhibitory peptide (Fig. 9).

### [Example 10] Measurement of Renin Concentration with Addition of Renin Inhibitor III by Sandwich ELISA Using RREN21E10-19 in Combination with Our Company Antibody rREN-I-115 or RREN33H11

### (1) Measurement of Renin Concentration

To an assay plate, 2 µg/mL of a solution of our company antibody rREN-I-115 or RREN33H11 diluted with PBS was added, followed by immobilization. Subsequent to washing three times with PBS-T, 1% BSA-PBS was added to carry out blocking. Subsequent to washing three times with PBS-T, a mixture in equal amounts of 200 ng/ml of a solution of recombinant mature renin diluted with PBS and each of 2000, 400, 80, 16, and 3.2 ng/mL of a solution of Renin Inhibitor III was added to the assay plate, followed by a primary reaction. Subsequent to washing three times with PBS-T, biotinylated labeled RREN21E10-19 diluted with 1% BSA-PBS was added to the assay plate, followed by a secondary reaction. Subsequent to washing three times with PBS-T, a solution of POD-labeled streptavidin diluted with 1% BSA-PBS was added to carry out a tertiary reaction. Subsequent to washing three times with PBS-T, TMB was added to carry out a chromogenic reaction. The 0.5 M H2SO4 was added to stop the chromogenic reaction, and the absorbance at 405 nm was measured.

### (2) Measurement of Renin Activity

To a white plate, 160 µL of assay buffer (50 mM Tris, 0.1 M NaCl, 10 mM EDTA) was added. Next, 20 µL of 20 µM renin fluorescent substrate (DMSO, Cayman Chemical) was added, and 20 µL of a mixture in equal amounts of a solution of recombinant mature renin and a solution of Renin Inhibitor III (same sample as that used in the concentration measurement) and a renin concentration calibrator were further added. Reaction was carried out at 37°C for 1 hour to detect fluorescence at 490 nm (excitation light: 340 nm).

As a result, as in the case of adding aliskiren, the combination with RREN33H11 showed no correlation with renin activity, but the combination with rREN-I-115 strongly correlated with renin activity (Fig. 10).

### [Example 11] Measurement of Prorenin Concentration with Addition of Aliskiren by Sandwich ELISA Using Combination of Our Company Antibodies rREN-I-115 and RREN21E10-19

To an assay plate, 2 µg/mL of a solution of our company antibody rREN-I-115 or RREN33H11 diluted with PBS was added, followed by immobilization. Subsequent to washing three times with PBS-T, 1% BSA-PBS was added to carry out blocking. Subsequent to washing three times with PBS-T, a mixture in equal amounts of each of 200, 50, 8, and 0.4 ng/ml of a solution of recombinant prorenin diluted with PBS and 10 µg/mL of aliskiren solution was added to the assay plate, followed by a primary reaction. Subsequent to washing three times with PBS-T, biotinylated labeled RREN21E10-19 diluted with 1% BSA-PBS was added to the assay plate, followed by a secondary reaction. Subsequent to washing three times with PBS-T, a solution of POD-labeled streptavidin diluted with 1% BSA-PBS was added to carry out a tertiary reaction. Subsequent to washing three times with PBS-T, TMB was added to carry out a chromogenic reaction. The 0.5 M H2SO4 was added to stop the chromogenic reaction, and the absorbance at 405 nm was measured.

As a result, it was confirmed that sandwich ELISA using the combination of rREN-I-115 and RREN21E10-19 also did not measure prorenin bound to aliskiren (Fig. 11).

### [Example 12] Identification of Epitope

### (1) Preparation of Renin Cleaved Fragments

The renin gene sequence of full length was appropriately fragmented (A, B, C, D, E, A3, A13, and A23 in the figure) to prepare renin fragment sequences added at the end with a restriction enzyme digestion site (Fig. 12). The restriction enzyme was used to introduce these renin fragment sequences into a GST expression vector to prepare a renin cleaved fragment expression vector fused with GST at the N-terminus. Gene delivery of the prepared expression vector was carried out into E. coli DH5α to amplify the plasmid. The resultant plasmid was introduced into E. coli BL21, followed by culturing in LB medium overnight with shaking. After that, the bacterial solution was diluted with LB medium to give a 10-fold dilution, followed by culturing with shaking for 1 hour. Expression was induced with 1 mM IPTG, followed by further culturing with shaking for 3 hours. The cells were recovered by centrifugation at 15000 rpm at room temperature for 1 minute. To the cells, 300 µL of PBS containing 0.01% Tween 20 was added, and the suspension was sonicated for 15 minutes. The sonicated solution was centrifuged at 15000 rpm at room temperature for 10 minutes, and the supernatant was recovered to prepare a solution of cleaved fragments.

### (2) Identification of Epitope by Western Blotting

To the solution of cleaved fragments, a half amount of 3 × SDS-PAGE sample buffer was added, followed by heat denaturing at 96°C for 5 minutes. To SDS-PAGE gel (Mini PROTEAN TGX Precast Gel, 4-20%, Bio rad), 7.5 µL (of 25 µL cultured) of the prepared sample was added, followed by electrophoresis at a constant voltage of 200 V for 30 minutes. The SDS-PAGE gel was transferred on a PVDF membrane using i-Blot (Thermo scientific), and the PVDF membrane was immersed in 1% skim milk-containing PBS to carry out blocking. The PVDF membrane was immersed in 1% skim milk-containing PBS containing 1 ug/mL of RREN63A8 and anti-GST antibody (GST 2-1), followed by shaking to carry out a primary reaction. The PVDF membrane was washed three times with PBS-T for 5 minutes and immersed in a solution of POD-labeled anti-mouse antibody diluted 1000 times with 1% skim milk-containing PBS, followed by shaking to carry out a secondary reaction. The PVDF membrane was washed three times with PBS containing Tween 20 for 5 minutes, and a luminescent substrate (ECL Prime, GE Healthcare) was added to the PVDF membrane. The excess liquid was removed to confirm the luminescence with LAS 500 (GE Healthcare).

As a result, RREN63A8 recognized REN-A, -A13, -A23, and -A3, but did not recognize REN-B, -C, -D, and -E (Fig. 13). Since RREN63A8 recognized REN-A3, it was shown to recognize the range of the 57 amino acids from the 30th to 86th amino acids of the renin amino acid sequence. Furthermore, since it did not recognize REN-D, the possibility was also suggested of recognizing the range of the 28 amino acids from the 30th to 57th amino acids of the renin amino acid sequence.

### (3) Test on Inhibition between Antibodies

Three types of our company antibodies (rREN-d-104-1, rREN-I-115, and RREN63A8) diluted with PBS to 2 µg/mL were added to an assay plate, followed by immobilization. Subsequent to washing three times with PBS-T, 1% BSA-PBE was added to the assay plate to carry out blocking. Subsequent to washing three times with PBS-T, a mixture in equal amounts of 200 ng/mL of renin diluted with 1% BSA-PBS and 800 µg/mL of the three types of our company antibodies diluted with 1% BSA-PBS or 1% BSA-PBS was added to the assay plate, followed by a primary reaction. Subsequent to washing three times with PBS-T, 1 µg/ml of biotinylated RREN21E10-19 diluted with 1% BSA-PBS was added, followed by a secondary reaction. Subsequent to washing three times with PBS-T, a solution of POD-labeled streptavidin diluted with 1% BSA-PBS was added to carry out a tertiary reaction. Subsequent to washing three times with PBS-T, TMB was added to carry out a chromogenic reaction. The 0.5 M H2SO4 was added to stop the chromogenic reaction, and the absorbance at 405 nm was measured.

As a result, the reaction was inhibited by an inhibitory antibody added together with an antigen for all combinations of the three types of antibodies (Fig. 14). This indicated that rREN-d-104-1 and rREN-I-115 recognized the same epitope as that of RREN63A8.

### [Example 131 Measurement of Concentration of VTP-27999-Added Renin by Sandwiching Using RREN21E10-19 in Combination with Our Company Antibody rREN-I-115 or RREN33H11

### (1) Measurement of Renin Concentration

To an assay plate, 2 µg/mL of a solution of our company antibody rREN-I-115 or RREN33H11 diluted with PBS was added, followed by immobilization. Subsequent to washing three times with PBS-T, 1% BSA-PBS was added to carry out blocking. Subsequent to washing three times with PBS-T, a mixture in equal amounts of 200 ng/ml of a solution of recombinant renin diluted with PBS and each of 100, 20, 4, 0.8, 0.16, and 0 nM VTP-27999 solutions was added to the assay plate, followed by a primary reaction. Subsequent to washing three times with PBS-T, biotinylated labeled RREN21E10-19 diluted with 1% BSA-PBS was added to the assay plate, followed by a secondary reaction. Subsequent to washing three times with PBS-T, a solution of POD-labeled streptavidin diluted with 1% BSA-PBS was added to carry out a tertiary reaction. Subsequent to washing three times with PBS-T, TMB was added to carry out a chromogenic reaction. The 0.5 M H2SO4 was added to stop the chromogenic reaction, and the absorbance at 405 nm was measured.

### (2) Measurement of Renin Activity

To a white plate, 160 µL of assay buffer (50 mM Tris, 0.1 M NaCl, 10 mM EDTA) was added. Next, 20 µL of 20 µM renin fluorescent substrate (DMSO, Cayman Chemical) was added, and 20 µL of a mixture in equal amounts of a solution of recombinant renin and a solution of VTP-27999 (same sample as that used in the concentration measurement) and a renin concentration calibrator were further added. Reaction was carried out at 37°C for 1 hour to detect fluorescence at 490 nm (excitation light: 340 nm).

As a result, as in the case of adding aliskiren, the combination with RREN33H11 showed no correlation with renin activity, but the combination with rREN-I-115 strongly correlated with renin activity (Fig. 15).

### [Example 14] Measurement of Concentration of Aliskiren-Added Renin by Sandwiching Using Combination of Our Company Antibodies rREN-d-104-1 and RREN21E10-19

### (1) Measurement of Renin Concentration

To an assay plate, 2 ug/mL of a solution of our company antibody RREN63A8 diluted with PBS was added, followed by immobilization. Subsequent to washing three times with PBS-T, 1% BSA-PBS was added to carry out blocking. Subsequent to washing three times with PBS-T, a mixture in equal amounts of 400 ng/ml of a solution of recombinant renin diluted with PBS and each of 200, 40, 8, 1.6, and 0.32 ng/mL of aliskiren solution was added to the assay plate, followed by a primary reaction. Subsequent to washing three times with PBS-T, biotinylated labeled RREN21E10-19 diluted with 1% BSA-PBS was added to the assay plate, followed by a secondary reaction. Subsequent to washing three times with PBS-T, a solution of POD-labeled streptavidin diluted with 1% BSA-PBS was added to carry out a tertiary reaction. Subsequent to washing three times with PBS-T, TMB was added to carry out a chromogenic reaction. The 0.5 M H2SO4 was added to stop the chromogenic reaction, and the absorbance at 405 nm was measured.

### (2) Measurement of Renin Activity

To a white plate, 160 µL of assay buffer (50 mM Tris, 0.1 M NaCl, 10 mM EDTA) was added. Next, 20 µL of 20 µM renin fluorescent substrate (DMSO, Cayman Chemical) was added, and 20 µL of a mixture in equal amounts of a solution of recombinant renin and a solution of aliskiren (same sample as that used in the concentration measurement) and a renin concentration calibrator were further added. Reaction was carried out at 37°C for 1 hour to detect fluorescence at 490 nm (excitation light: 340 nm).

As a result, as in the case of rREN-I-115, sandwich ELISA using the combination with clone rREN-d-104-1 selected with the reactivity to renin in the presence of aliskiren as an index showed correlation between renin concentration and renin activity (Fig. 16).

### [Example 15] Measurement of Concentration of Renin in Aliskiren-Added Specimen

To an assay plate, 5 ug/mL of a solution of commercially available antibody 12-12 or our company antibody rREN-I-115, RREN33H11, or rREN-d-104-1 diluted with PBS was added, followed by immobilization. Subsequent to washing three times with PBS-T, 1% BSA-PBS was added to carry out blocking. Subsequent to washing three times with PBS-T, aliskiren was added to a concentration of 1000 nM to 2 types of commercially available human plasma for which mature renin contained was already priced, and the mixture left standing at room temperature for about 1 hour was diluted with a specimen diluting solution (manufactured by FUJIREBIO Inc.) to give a 5-fold dilution, followed by addition to the assay plate to carry out a primary reaction. Subsequent to washing three times with a wash solution (manufactured by FUJIREBIO Inc.), biotinylated labeled 11-6 or biotinylated labeled RREN21E10-19 diluted to 1 µg/mL with a Tris buffer solution containing 2% BSA was added to the assay plate to carry out a secondary reaction. Subsequent to washing three times with a wash solution (manufactured by FUJIREBIO Inc.), a solution of ALP labeled streptavidin diluted to 0.01 µg/mL with a Tris buffer solution containing 2% BSA was added to carry out a tertiary reaction. Subsequent to washing three times with a wash solution (manufactured by FUJIREBIO Inc.), a substrate solution (manufactured by FUJIREBIO Inc.) was added, followed by reaction at 37°C for 5 minutes to detect chemiluminescence.

As a result, as in the case of adding aliskiren to recombinant renin, the combination of commercially available antibodies 12-12 and 11-6 or the combination of our company antibodies RREN33H11 and RREN21E10-19 showed almost no change in renin concentration with the addition of aliskiren. However, the combination containing rREN-I-115 or rREN-d-104-1 showed a decrease in renin concentration with the addition of aliskiren (Fig. 17).

This indicated that rREN-I-115 and rREN-d-104-1 did not recognize renin having no activity due to the binding of aliskiren.

### [Industrial Applicability]

As has been described above, the present invention makes it possible to efficiently measure the concentration of active renin which is in correlation with renin activity by use of an active renin-specific antibody. Since renin activity is associated with diseases such as primary aldosteronism, the present invention can greatly contribute not only to research use but also to diagnosis of diseases.

## Claims

1. A monoclonal antibody **characterized by** the following (a) to (c);
(a) showing higher reactivity to mature renin and open structure prorenin, both of which are not bound with a renin inhibitor, than to mature renin and open structure prorenin, both of which are inactivated by binding of a renin inhibitor,
(b) binding to a peptide composed of 30th to 86th amino acids of an amino acid sequence set forth in SEQ ID NO: 1, and
(c) not binding to a peptide composed of 58th to 170th amino acids of an amino acid sequence set forth in SEQ ID NO: 1.

2. A method for measuring a concentration of renin in a sample using an antibody, wherein the antibody is the monoclonal antibody according to claim 1.

3. A kit for use in the method according to claim 2, comprising at least the monoclonal antibody according to claim 1.

4. A composition for detecting renin in a sample, comprising the monoclonal antibody according to claim 1.

## Patentansprüche

1. Monoklonaler Antikörper, **gekennzeichnet durch** die folgenden (a) bis (c);
(a) Zeigen einer höheren Reaktivität gegenüber reifem Renin und Prorenin mit offener Struktur, die beide nicht mit einem Renin-Inhibitor gebunden sind, als gegenüber reifem Renin und Prorenin mit offener Struktur, die beide durch Bindung eines Renin-Inhibitors inaktiviert sind,
(b) Bindung an ein Peptid, bestehend aus den 30. bis 86. Aminosäuren einer in SEQ ID NO: 1 dargestellten Aminosäuresequenz, und
(c) keine Bindung an ein Peptid, bestehend aus den 58. bis 170. Aminosäuren einer in SEQ ID NO: 1 dargestellten Aminosäuresequenz.

2. Verfahren zum Messen einer Konzentration von Renin in einer Probe unter Verwendung eines Antikörpers, wobei der Antikörper der monoklonale Antikörper nach Anspruch 1 ist.

3. Kit zur Verwendung in dem Verfahren nach Anspruch 2, umfassend mindestens den monoklonalen Antikörper nach Anspruch 1.

4. Zusammensetzung zum Nachweis von Renin in einer Probe, umfassend den monoklonalen Antikörper nach Anspruch 1.

## Revendications

1. Anticorps monoclonal **caractérisé par** les points suivants (a) à (c) :
(a) présentation d'une réactivité supérieure à la rénine mûre et à la prorénine à structure ouverte, qui ne sont toutes deux pas liées à un inhibiteur de rénine, par rapport à la rénine mûre et à la prorénine à structure ouverte, qui sont toutes deux inactivées par liaison d'un inhibiteur de rénine,
(b) liaison à un peptide composé des 30^{e} à 86^{e} acides aminés d'une séquence d'acides aminés établie dans SEQ ID N° : 1, et
(c) absence de liaison à un peptide composé des 58^{e} à 170^{e} acides aminés d'une séquence d'acides aminés établie dans SEQ ID N° : 1.

2. Procédé de mesure d'une concentration en rénine dans un échantillon utilisant un anticorps, dans lequel l'anticorps est l'anticorps monoclonal selon la revendication 1.

3. Kit destiné à être utilisé dans le procédé selon la revendication 2, comprenant au moins l'anticorps monoclonal selon la revendication 1.

4. Composition pour détecter de la rénine dans un échantillon, comprenant l'anticorps monoclonal selon la revendication 1.
